(19) Europäisches Patentamt
European Patent Office
Office européen des brevets

(11) Veröffentlichungsnummer: **0 017 708**
**B1**

(12) **EUROPÄISCHE PATENTSCHRIFT**

(45) Veröffentlichungstag der Patentschrift: **18.01.84**

(21) Anmeldenummer: **80100556.2**

(22) Anmeldetag: **04.02.80**

(51) Int. Cl.³: **C 13 K 3/00, C 13 K 11/00, C 12 P 7/42, C 12 P 19/02**

(54) **Verfahren zur enzymatischen Glucoseoxidation.**

(30) Priorität: **22.03.79 DE 2911192**

(43) Veröffentlichungstag der Anmeldung:
**29.10.80 Patentblatt 80/22**

(45) Bekanntmachung des Hinweises auf die Patenterteilung:
**18.01.84 Patentblatt 84/3**

(84) Benannte Vertragsstaaten:
**AT BE CH DE FR GB IT LU NL SE**

(56) Entgegenhaltungen:
**DE - A - 1 517 814**
**DE - A - 2 636 206**

**CHIMIA, Band 29, Nr. 3, März 1975, Seiten 123-131, V. KRASNOBAJEW et al.: "Verwendungsmöglichkeiten von PAG-Copolymer-immobilisierten Enzymen in der Stärkeindustrie"**

(73) Patentinhaber: **C.H. BOEHRINGER SOHN**
**Postfach 200**
**D-6507 Ingelheim am Rhein (DE)**

(72) Erfinder: **Hartmeier, Winfried, Dr.**
**D-6507 Leipziger Strasse 3**
**Ingelheim (DE)**

**EP 0 017 708 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

# Verfahren zur enzymatischen Glucoseoxidation

Die Erfindung betrifft ein Verfahren zur enzymatischen Glucoseoxidation mit Hilfe von partikelgebundener wasserunlöslicher Glucoseoxidase-Katalase mit bestimmter Katalase-Mindestaktivität bei niedrigen Temperaturen.

Es ist bereite bekannt, Glucose enzymatisch in begasten Rührreaktoren mittels eines Enzymgemische aus Glucoseoxidase (GOD) und Katalase (KAT) zu oxidieren. Das Enzymgemisch (GOD-KAT) bewirkt dabei eine Oxidation von Glucose zu Glucono-$\delta$-Lacton, das in wäßriger Lösung in der Regel spontan oder katalysiert durch Lactonase in Gluconsäure übergeht.

$$\text{Glucose} + 1/2\ O_2 \rightarrow \text{Glucon-}\delta\text{-Lacton} \rightleftharpoons \text{Gluconsäure}$$

Im einzelnen bewirken die beiden Einzelenzyme GOD und KAT den Reaktionsablauf in folgenden hintereinander geschalteten Teilschritten

$$\text{Glucose} + O_2 + H_2O \xrightarrow{\text{GOD}} \text{Gluconsäure} + H_2O_2$$

$$H_2O_2 \xrightarrow{\text{KAT}} H_2O_2 + 1/2\ O_2$$

Bei Vorhandensein von Katalase, wie es in vielen technischen GOD-Präparaten gegeben ist, wird das intermediär gebildete Wasserstoffperoxid gespalten, so daß es in der Regel zu keiner merklichen Anhäufung von Wasserstoffperoxid im Reaktionsmedium kommt. Die Reaktion verläuft somit nach außen hin meist nach der erstgenannten Gesamtgleichung. In der Fach- und Patentliteratur wird dem Katalasegehelt indes wenig Beachtung geschenkt, vielmehr wird der Katalaseanteil oft nicht einmal erwähnt, sondern es wird nur von Glucoseoxidase gesprochen.

Eine industrielle Nutzung löslicher GOD-KAT-Gemische erfolgt bereits seit langem in der Lebensmittelindustrie zur Entfernung von Glucosespuren oder Sauerstoffspuren aus Nahrungsmitteln oder Getränken. So wird lösliche GOD-KAT z.B. Eiprodukten zugemischt, um Spuren von Glucose daraus zu entfernen. Ein weiteres häufiges Anwendungsgebiet ist der Softdrink-Sektor. Dort dient das dem Getränk zugesetzte GOD-KAT-Gemisch der Sauerstoffentfernung und damit der Verhinderung von Oxidationsschäden. Auch als analytisches Hilfsmittel, z.B. zur Glucosebestimmung hat GOD weite Verbreitung gefunden.

Im Gegensatz zu den genannten Einsatzgebieten hat für die technische Glucoseoxidation GOD-KAT bisher noch keine oder nur vereinzelte praktische Anwendung gefunden. Aus der US—PS—3 050 444 ist zwar ein Verfahren bekannt, bei dem in Invertzucker (=Glucose+Fructose-Mischungen) enthaltene Glucose mit Hilfe löslicher GOD-KAT oxidiert wird. Die gebildete Gluconsäure läßt sich z.B. durch Fällung—z.B. als Calciumsalz—wesentlich leichter von der Fructose abtrennen als die ursprünglich vorhandene Glucose. Das Verfahren wurde in den 60er Jahren in den USA großtechnisch praktiziert. Infolge des relativ hohen Preises für GOD-KAT ist es jedoch heute nicht mehr konkurrenzfähig.

Um den hohen Enzympreis zu kompensieren, wurde in der DE—AS—20 03 732 vorgeschlagen, die GOD durch Elektrodialyse aus der Reaktionslösung wiederzugewinnen und dann wiederholt in löslicher Form einzusetzen. Lt. dieser DE-AS kennten mit 0,688 g handelsüblicher löslicher GOD 830 g Natriumgluconat hergestellt werden, d.h. mit 1 g löslicher GOD können nach diesem Verfahren 1206 g Natriumgluconat erzielt werden. Ein beträchtlicher Nachteil dieses Verfahrens ist es aber, daß die Elektrodialyse eine komplizierte Apparatur erfordert, die sich im rauhen Praxisbetrieb großtechnisch kaum beherrschen läßt. Die erforderlichen Mehrmembran-Dialyseapparate sind anfällig gegen Beschädigungen und Störungen.

Da immobilisierte (trägerfixierte) Enzympräparate und darunter auch immobilisierte GOD und KAT seit Jahren zum Stand der Technik gehören, erscheint ferner der Gedanke, immobilisierte Präparate auch zur enzymatischen Glucoseoxidation einzusetzen, naheliegend zu sein. Jedoch konnte bisher kein wirtschaftliches und großtechnisch einsetzbares Verfahren auf der Basis immobilisierter GOD und KAT entwickelt werden. Vielmehr ist aus der Vielzahl von Publikationen auf diesem Gebiet zu entnehmen, daß die Haltbarkeit solcher Präparate unter praxisüblichen Bedingungen bisher nur wenige Tage beträgt. Publikationen dieser Art sind:

R. Carter, J. E. Prenosil & J. R. Bourne: A Deactivation Study of the Immobilized Glucose Oxidase/Catalase System.—First Europ. Congr, Biotechnol.,—Preprints, Part 1, S. 107—108, Interlaken, 1978.

K. Buchholz & M. Reuss: Kopplung von Stofftransport, Reaktion und Desaktivierung bei trägergebundener Glucoseoxidase und Katalase.—Chimia 31, 27—30 (1977).

K. Buchholz & B. Gödelmann: Macrokinetics and Operational Stability of Immobilized Glucose Oxidase and Catalase.—Biotechnol. Bioengng. *20*, 1201—1220 (1978).

K. B. Ramachandran & D. D. Perlmutter. Effects of Immobilization of the Kinetics of Enzym-Catalyzed Reactions. I. Glucose Oxidase in a Recirculated Reactor System.—Biotechnol. Bioengng. *18*, 669—684 (1976).

S. Krishnaswamy & J. R. Kittrell: Deactivation Studies of Immobilized Glucose Oxidase.—Biotechnol. Bioengng. *20*, 821—835 (1978).

Eine gewisse, aber nach wie vor unzureichende Verbesserung der Stabilität der immobilisierten GOD und KAT-Präparate konnte gemäß einigen der obengenannten Publikationen zusätzlich durch Verwendung von die Peroxidspaltung fördernden Trägermaterialien—wie z.B. Manganoxid der Rutheniumverbindungen—erreicht werden. Die Halbwertzeit, das ist die bis zur Aktivitätsabnahme auf die Hälfte des ursprünglichen Wertes erforderliche Zeit, ist auch bei diesen Präparaten unter den vorgeschlagenen und in der Biotechnologie meist angewandten Reaktionsbedingungen bei 25 bis 35°C auf wenige Tage beschränkt. Die Mitverwendung anorganischer Katalysatoren—wie z.B. Manganoxid—ist auch lebensmittelrechtlich bedenklich.

Aufgabe der vorliegenden Erfindung ist es, die nach dem bisherigen Stand der Technik erreichbare Gesamt-Glucoseumsatzmenge pro Gewichtseinheit eingesetzter GOD zu steigern. Laut DE—AS—20 03 732 lag diese insgesamt umgesetzte Glucosemenge bisher bei ca. 1200 Gramm Glucose pro 1 Gramm handelsüblicher GOD-Zubereitung. Weiterhin ist Aufgabe der Erfindung, das Verfahren in apparatetechnisch einfacher Weise und lebensmittelrechtlich unbedenklich zu gestalten.

Es wurde nun gefunden, daß die oben geschilderte Aufgabe gelöst werden kann, wenn man zur Glucoseoxidation ein immobilisiertes Glucoseoxidase-Katalasepräparat einsetzt, bei dem beide Enzyme in oder an jeweils demselben Partikel gebunden sind und das dadurch gekennzeichnet ist, daß die Katalaseaktivität—ausgedrückt in Bakereinheiten—mindestens ein Sechstel der Glucoseoxidaseaktivität—ausgedrückt in Saretteinheiten—beträgt, und daß die Oxidationsreaktion mittels der vorstehend beschriebenen partikelgebundenen Enzyme bei Temperaturen unterhalb 15°C, insbesondere bei Temperaturen zwischen 10°C und dem Gefrierpunkt des Reaktionsgemischs durchzuführet wird. Diese für biotechnische Reaktionen ungewöhnlich tiefe Temperaturführung gewährleistet eine hohe Wirtschaftlichkeit.

Die Herstellung des verfahrensgemäß verwendeten Präparats (d.h. die Enzym-Fixierung an wasserunlösliche Trägerpartikel) kann nach einem an sich bekannten Verfahren erfolgen. Indes sind Präparate, wie sie z.B. in den schon zitierten Arbeiten von Buchholz und Mitarbeiten verwendet wurden, ungeeignet, weil dort Katalase und Glucoseoxidase an jeweils unterschiedliche Partikel gebunden waren. Für die Durchführung des erfindungsgemäßen Verfahrens ist es vielmehr unerläßlich, daß Glucoseoxidase und Katalase in oder an jeweils ein und demselben wasserunlöslichen Partikel gebunden sind. Dabei kann die Bindung der beiden Enzyme gleichzeitig oder nacheinander erfolgen. Wichtig ist, daß Glucoseoxidase und Katalase im eingesetzten Enzympräparat in größtmöglicher Nähe nebeneinander vorliegen. Gut geeignet für die Immobilisierung ist z.B. das Verfahren gemäß DE—AS—26 36 206. Hiernach werden die Enzyme an Trägerpartikel aus hochmolekularporösem, formaldehydgehärteten Protein (z.B. Gelatine), welches eine Wasseraufnahmefähigkeit vom 2-8-fachen seines Trockengewichts aufweist, gebunden, wobei die Fixierung vorzugsweise mittels Glutardialdehyd erfolgt. Als besonders günstig haben sich Teilchengrößen zwischen 10 und 100 $\mu$m erwiesen.

Wie bereits erwähnt muß die Katalaseaktivität in den enzymatisch aktiven Partikelnausgedrückt in Bakereinheiten—mindestens ein Sechstel, vorzugsweise jedoch mindestens ein Viertel der Glucoseoxidaseaktivität—ausgedrückt in Saretteinheiten—betragen. Während nach bisheriger Erkenntnis auch weniger als der genannte Katalaseanteil ausreicht, um eine Anhäufung von Wasserstoffperoxid im Reaktionsmedium zu verhindern, wurde nun gefunden, daß eine optimale Glucoseumsatzmenge nur erreicht wird, wenn der genannte Mindest-Katalaseanteil vorliegt. Der Katalaseanteil kann natürlich gewünschtenfalls wesentlich über dem genannten Mindestmaß liegen. Auf wirtschaftlichen Erwägungen wird er jedochausgedrückt in Bakereinheiten—normalerweise nicht höher liegen als der Glucoseoxidasegehalt—ausgedrückt in Saretteinheiten.

Als Trägerstoffe für die gebundenen Enzyme können neben üblichen Trägermaterialien gewünschtenfalls auch anorganische peroxidspaltende Katalysatoren eingesetzt werden. Das erfindungsgemäße Verfahren ist jedoch nicht an die Verwendung von immobilisierten Enzymen auf derartigen Trägerstoffen gebunden. Aus lebensmittelrechtlichen Überlegungen muß in der Regel auf die Verwendung von Enzymen mit derartigen Trägerstoffen verzichtet werden.

Gemäß einer weiteren Ausbildung der Erfindung können neben Glucoseoxidase und Katalase auch noch andere Enzyme in löslicher oder immobilisierter Form im erfindungsgemäßen Verfahren eingesetzt werden. Zum Beispiel kann durch amylolytische Enzyme Glucose aus Dextrinen bzw. Stärke freigesetzt werden, die dann durch das Glucoseoxidase-Katalase-System weiter umgesetzt wird.

In einer bevorzugten Ausführungsform des erfindungsgemäßen Verfahrens werden 500 bis 5000 Sarett-Einheiten Glucoseoxidase und 200 bis 2000 Baker-Einheiten Katalase pro Liter Reaktionsflüssigkeit in der oben näher erläuterten immobilisierten Form (d.h. an wasserunlösliche Partikel aus organischem Trägermaterial gebunden) eingesetzt.

Die Glucosekonzentration im Reaktionsmedium kann innerhalb weiter Grenzen schwanken. Sie liegt in der Regel zwischen 0 und 40 g pro 100 ml. Höhere Konzentrationen sind zwar möglich, sie mindern jedoch den Sauerstoffübergang und damit auch die von der Sauerstoffkonzentration im Reaktionsmedium

abhängige Glucose-Umsatzrate zunehmend mit ansteigender Glucosekonzentration.

Der Rührreaktor wird—wie bei solchen Verfahren allgemein üblich—mit Sauerstoff oder einem sauerstoffhaltigen Gas—z.B. Luft—begast. Dabei beträgt die Begasungsrate normalerweise 0,1 bis 2,0 Volumen Gas unter Normaldruck pro 1 Volumen Reaktionsmedium und Minute. Zum Beispiel werden 1000 Liter Fermentationsflüssigkeit mit 1 $Nm^3$ Luft pro Minute begast. Eine Sauerstoffversorgung durch Zudosierung von Wasserstoffperoxid erwies sich für die Durchführung des erfindungsgemäßen Verfahrens als ungeeignet, weil hierbei eine zu starke Enzyminaktivierung stattfindet.

Der pH-Wert der Reaktionsflüssigkeit muß, wie bei biotechnischen Reaktionen üblich, in einem für die Enzyme günstigen Bereich gehalten werden, das ist in der Regel pH 4—7. Als zweckmäßig hat sich z.B. eine automatische pH-Stat-Titration mit NaOH oder KOH auf pH 5,5 erwiesen. Je nach Herkunft der verwendeten GOD und KAT (aus Schimmelpilzen, Bakterien, Rinderleber o.ä.) kann der günstigste pH Wert innerhalb der angegebenen Grenzen variieren. Er kann jedoch in einer dam Fachmann vertrauten Weise durch Versuche leicht ermittelt werden.

Der besagte Rührreaktor sollte—sofern die Enzympartikel zum Aufsteigen und Anbacken neigen—zur Verhinderung des Anbackens von Enzympertikeln im Reaktorkopfraum intermittierend oder ständig gespült oder gewischt werden. Dies dann z.B. in einfacher Weise dadurch geschehen, daß die Reaktionsflüssigkeit teilweise oder ganz umgepumpt und z.B. schräg von oben gegen die Wandung im Reaktionskopfraum gespritzt wird, z.B. durch eine gelochte Ringleitung. Die Fermentationsflüssigkeit kann dazu mit oder ohne Enzympartikel rundgepumpt werden. Bevorzugt erfolgt die Rundpumpung ohne Enzympartikel, um diese vor unerwünschten Scherbelastungen zu bewahren, indem man z.B. vor die Absaugleitung ein Filter oder Sieb anbringt.

In einer bevorzugten Ausführungsform wird das erfindungsgemäße Verfahren in einem Reaktor durchgeführt, der im unteren Teil eine sieb-, membran- oder filterbedeckte Absaugvorrichtung und im Kopfraum eine Ringsprühvorrichtung besitzt. Abbildung 1 zeigt eine beispielhafte Ausführung eines solchen begasten Rührreaktors. In dieser Abbildung bedeuten:

1=Reaktorfüllung; 2=Rührmotor; 3=Rührer; 4=Filterschicht, Membran oder Sieb mit Stützvorrichtung; 5=Temperiermantel; 6=Pumpe; 7=Ringleitung mit Sprühdüsen; 8=Begasungsring; 9=Begasungszufuhr; 10=Prallblech zur Verhinderung des Mitdrehens der Fermentationsbrühe; 11=pH-Meß und automatische Titriervorrichtung; 12=Dreiwegehahn und Ablauf.

Die in Abbildung 1 beispeilhaft gezeigte filter-, membran- oder siebbedeckte Absaugvorrichtung am Fermenterboden hat auch dem Vorteil, daß die gesamte umgesetzte Fermentationsflüssigkeit nach beendeter Reaktion abgepumpt und über den Dreiweghahn ihrer weiteren Verwendung—z.B. Fällung o.ä.—zugeführt werden kann. Die partikelgebundenen Enzyme verbleiben im Reaktor. Sie können gewünschtenfalls desinfiziert, gespült und wiederverwendet werden, ohne aus dem Reaktor entnommen werden zu müssen.

Die Apparatur zur Durchführung des erfindungsgemäßen Verfahrens kann natürlich in weitem Maß variiert werden.

Zum Beispiel kann ein Anhaften von Enzympartikeln an den Fermenterkopfraumwandungen auch durch mechanische Wischer verhindert werden. Beispielsweise bei spezifisch sehr schweren Enzympartikeln, die nicht zum Hochsteigen an Reaktorwandungen neigen, kann diese Maßnahme des Freihaltens der Reaktorkopfwandungen von anhaftenden Enzympartikeln natürlich entfallen.

Das erfindungsgemäße Verfahren dient bevorzugt der Glucoseoxidation zum Zweck der Glucon-$\delta$-Lacton-, Gluconat-, Gluconsäure- und/oder Fructosegewinnung. Der Fructosegewinnung kann das Verfahren dadurch dienen, daß Glucose in Gemischen aus Glucose und Fructose (z.B. Invertzucker, Isomerose) oxidiert wird. Die gebildete Gluconsäure bzw. das gebildete Gluconat läßt sich dann durch Fällung (z.B. als Ca-Gluconat) oder durch Ionenaustausch wesentlich einfacher von der verbleibenden Fructose abtrennen als die ursprünglich vorhandene Glucose.

Ein beträchtlicher Vorteil des erfindungsgemäßen Verfahrens ist es, daß die eingesetzten Enzympartikel nicht schon nach wenigen Tagen inaktiviert sind, sondern über Monate hinweg verwendbar blieben. Auch die Infektionsgefahr ist beim erfindungsgemäßen Verfahren infolge der für Mikroorganismen ungünstig tiefen Temperatur gut beherschbar. Die mit einem Gramm handelsüblichem GOD-KAT-Gemisch erzielbare gesamte Glucoseumsatzmenge steigt auf 10 kg und mehr an und übertrifft damit den gegebenen Stand der Technik erheblich.

Die nachfolgenden Beispiele erläutern die Erfindung ohne sie zu beschränken:

Beispiel 1

1 kg Speisegelatine mit 80 Bloom wurden in 3 Liter entionisiertem Wasser durch Erwärmen auf 60°C unter Rühren gelöst. Unter weiterem schnellen Rühren wurden 60 ml 30%ige Formaldehydlösung zugesetzt. Nach ca. 4 Minuten weiterem Rühren bei 60°C geliert die Mischung. Sie wurde 24 Stunden bei Raumtemperatur stehen gelassen, dann grob zerkleinert und auf Hordenlechen bei 105°C im Trockenschrank getrocknet. Die Trockenpartikel wurden auf die gewünschte Feinheit

vermahlen und ergaben die Trägerstoffpartikel für die Enzymimmobilisierung.

Für die folgende Fixierung wurde als handelsübliche Glucoseoxidase-Katalase das unter dem Handelsnamen Glucox-R vertriebene Produkt der Fa. J & E. Sturge, Großbritannien, eingesetzt. Es hatte eine Glucoseoxidaseaktivität von 1580 Saretteinheiten pro ml und eine Katalaseaktivität von 540 Bakereinheiten pro ml. 100 ml dieses Produktes wurden mit 380 ml Wasser und 50 g der oben beschriebenen gehärteten Gelatine verrührt, die zuvor auf eine Korngröße von 20—50 µm vermahlen und ausgesiebt war. Dem Gemisch wurden sodann 700 ml Aceton und 30 ml 25%iger Glutardialdehyd beigemischt. Nach 2-stündigem Schütteln im Wasserbad bei 25°C wurden die nun enzymhaltigen Partikel abfiltriert und sorgfältig mit dest. Wasser gespült. Die Aktivität dieses Präparates betrug 1270 Saretteinheiten und 405 Bakereinheiten pro ml eingesetzter Glucox-R.

Die gesamte aus der Immobilisierung von 100 ml Glucox-R resultierende Menge fixiertes Enzym wurde in einem belüfteten Rührreaktor mit 50 Liter Substrat, das 5 kg Glucose und 5 kg Fructose enthielt, eingebracht. Der Reaktor wurde mit 50 Nl Luft pro Minute durch ein gelochtes Rohr begast und durch einen Blattrührer mit 560 Umdrehungen pro Minute gerührt. Durch automatische Titration mit 10%iger NaOH wurde der pH-Wert konstant auf 5,5±0,1 gehalten. Die Temperatur des Fermenterinhaltes wurde auf 2±1°C konstant gehalten. Ein Teil der Fermentationsflüssigkeit wurde ständig über den membranbedeckten Siebboden des Reaktors abgepumpt und dem Fermenter durch eine gelochte Ringleitung in der Weise wieder zugeführt, daß sie schräg von oben gegen die Wandung im Fermenterkopfraum gesprüht wurde. Dadurch wurde ein Ankleben von mit den Gasblasen hochgestiegenen Enzympartikeln an der Reaktor-Kopfwandung verhindert. Nach Umsatz sämtlicher Glucose zu Gluconat wurde die Fermentationsflüssigkeit über den am Fermentervoden angebrachten membranbedeckten Absaugstutzen abgesaugt. Die im Fermenter zurückbleibenden Enzympartikel wurden bei 2°C mit 0,5% eines von der Fa. C.H. Boehringer Sohn, Ingelheim, unter dem Handelsnamen Jodonal vertriebenen Desinfektionsmittel versetzt, das zuvor mit NaOH auf pH 5,0 eingestellt worden war. Vor Beginn der nächsten Glucoseumsetzung wurden die Enzympartikel mit Wasser durchgespült. Die erste Umsetzung war nach 12,2 Stunden beendet. Die folgenden Umsetzungen dauerten zwar zunehmend etwas länger, es konnten aber 200 Ansätze gefahren werden, bis die Umsatzdauer auf ca. 24 Stunden angewachsen war. Insgesamt wurden bei den 200 Ansätzen 1000 kg Glucose mit den ursprünglich eingesetzten 100 ml handelsüblicher Glucoseoxidase-Katalase umgesetzt. Eine Weiterführung darüberhinaus war möglich, da die Enzympartikel noch rund die Hälfte ihrer ursprünglichen Aktivität aufwiesen.

Beispiel 2

Als handelsübliche Glucoseoxidase-Katalase wurde das unter dem Handelsnamen Maxazym-GO-L-1500 von der Fa. Gist-Brocades, Delft, angebotene Produkt eingesetzt. Dieses Präparat wies eine Glucoseoxidaseaktivität von 1520 Saretteinheiten pro ml und eine Katalaseaktivität von 370 Bakereinheiten pro ml auf. 100 ml des Produktes wurden mit 400 ml dest. Wasser und 8 g Albumin aus Eiern verrührt. Der Lösung wurden unter Rühren 800 ml Isopropanol und 35 ml 25%iger Glutardialdehyd zugegeben. Nach 2 Stunden weiterem Rühren bei 25°C wurden die fixierten Enzympartikel durch Filtration abgetrennt und gründlich mit Wasser gespült. Die Aktivität in diesem fixierten Präparat betrug 860 Saretteinheiten und 206 Bakereinheiten pro ml des ursprünglich eingesetzten löslichen Enzyms.

Die gesamte aus der Immobilisierung von 100 ml löslichem GOD-KAT resultierende Enzymmenge wurde in einem belüfteten Rührreaktor mit 50 Liter 20%iger Glucoselösung eingesetzt. Der Reaktor wurde mit 50 Nl Luft pro Minute durch ein dicht über dem Reaktorboden angebrachtes gelochtes Ringrohr begast und durch einen Blattrührer mit 500 Umdrehungen pro Minute gerührt. Durch automatische Titration mit 10%iger KOH wurde der pH-Wert konstant auf 6,0±0,1 gehalten. Die Temperatur des Fermenterinhaltes wurde auf 8±1°C konstant gahelten. Ein Teil der Fermentationsflüssigkeit wurde ständig über dem mit einer Membran bedeckten Siebboden des Fermenters abgepumpt und über eine gelochte Ringleitung im Fermenterkopfraum in der Weise wieder zugeführt, daß sie schräg von oben gegen die Innenwandung des Fermenters gesprüht wurde. Dadurch wurde ein Aufsteigen und Ankleben von Enzympartikeln an den Wandungen verhindert. Nach Umsatz sämtlicher Glucose wurde die Fermentationsflüssigkeit über den membranbedeckten Siebboden abgesaugt. Die im Fermenter zurückbleibenden Enzympartikel wurden bei 2°C mit der 0,5%igen Lösung eines unter dem Namen Absonal von der Fa. C.H.

Boehringer Sohn vertriebenen Desinfektionsmittel versetzt, das zuvor auf pH 5,0 eingestellt worden war. Vor Beginn der nächsten Glucoseumsetzung wurden die Enzympartikel mit Wasser gespült. Die erste Umsetzung war nach 29,6 Stunden beendet. Bei weiteren 100 Ansätzen stieg die erforderliche Zeit für eine komplette Umsetzung der Glucose bis auf 68 Stunden an. Insgesamt wurden bei 100 durchgeführten Ansätzen mit den ursprünglich eingesetzten 100 ml handelsüblicher Glucoseoxidase-Katalase 1000 kg Glucose umgesetzt.

Glucoseoxidaseaktivität

Die Bestimmung der GOD-Aktivität erflogte

gemäß First Suppl. Food Chem. Codex, 2nd. Ed. S. 78—79, Verlag National Academy of Sciences, Washington, 1974. Die Aktivitätsangabe erfolgt in Saretteinheiten.

Katalaseaktivität

Die Bestimmung der KAT-Aktivität erfolgt gemäß First Suppl. Food Chem. Codex, 2nd. Ed. S. 67—68, Verlag National Academy of Sciences, Washington, 1974. Die Aktivitätsangabe erfolgt in Bakereinheiten.

## Patentansprüche

1. Verfahren zur enzymatischen Glucoseoxidation mit Hilfe partikelgebundener Glucoseoxidase-Katalase, dadurch gekennzeichnet, daß die Reaktion bei Temperaturen unterhalb von 15°C unter Verwendung eines immobilisierten Glucoseoxidase-Katalasepräparats, bei dem beide Enzyme jeweils in oder an demselben Partikel gebunden und die Katalaseaktivität—ausgedrückt in Bakereinheiten—mindestens ein Sechstel der Glucoseoxidaseaktivität—ausgedrückt in Saretteinheiten—beträgt, durchgeführt wird.

2. Verfahren nach Anspruch 1, dadurch gekennzeichnet, daß die Reaktion bei Temperaturen zwischen dem Gefrierpunkt der Reaktionsflüssigkeit und 10°C durchgeführt wird.

3. Verfahren nach einem der Ansprüche 1 oder 2, dadurch gekennzeichnet, daß 500 bis 5000 Sarett-Einheiten Glucoseoxidase und 200 bis 2000 Baker-Einheiten Katalase pro Liter Reaktionsflüssigkeit in partikelgebundener Form eingesetzt werden.

4. Verfahren nach Anspruch 1, dadurch gekennzeichnet, daß neben Glucoseoxidase und Katalase noch andere Enzyme in löslicher oder immobilisierter Form eingesetzt werden.

5. Verfahren nach einem der Ansprüche 1 bis 3, dadurch gekennzeichnet, daß es in einem Rührreaktor durchgeführt wird, der im unteren Reaktorteil, vorzugsweise am Boden, eine sieb-, membran- oder filterbedeckte Absaugvorrichtung aufweist, durch die partikelfreie Reaktionsflüssigkeit abgesaugt wird, welche dann durch Sprühen gegen die Wandungen im Reaktorkopfraum dem Reaktor wieder zugeführt wird.

## Revendications

1. Procédé pour l'oxydation enzymatique du glucose à l'aide de glucose oxydase-catalase liée à des particules, caractérisé en ce que la réaction est effectuée à des températures inférieures à 15°C en utilisant une préparation de glucose oxydase-catalase immobilisée, dans laquelle les deux enzymes sont liées à chaque fois dans ou sur la même particule et l'activité de la catalase—exprimée en unités Baker—est au moins un sixième de l'activité de la glucose oxydase-exprimée en unités Sarett.

2. Procédé suivant la revendication 1, caractérisé en ce que la réaction est effectuée à des températures entre le point de congélation du liquide réactionnel et 10°C.

3. Procédé suivant l'une des revendications 1 ou 2, caractérisé en ce qu'on utilise 500 à 5000 unités Sarett de glucose oxydase et 200 à 2000 unités Baker de catalase par litre de liquide réactionnel sous forme liée à des particules.

4. Procédé suivant la revendication 1, caractérisé en ce qu'à côté de la glucose oxydase et de la catalase on utilise encore d'autres enzymes sous forme soluble ou immobilisée.

5. Procédé suivant l'une des revendications 1 à 3, caractérisé en ce qu'il est effectué dans un réacteur à agitation qui présente à la partie inférieure du réacteur, de préférence au fond, un dispositif d'aspiration recouvert d'un tamis, d'une membrane ou d'un filtre, à travers lequel le liquide réactionnel exempt de particules est aspiré, lequel est ensuite ramené au réacteur par pulvérisation contre les parois dans l'espace de tête du réacteur.

## Claims

1. Process for enzymatic glucose oxidation using particle-bonded glucose oxidase/catalase, characterised in that the reaction is carried out at temperatures below 15°C using an immobilised glucose oxidase/catalase preparation wherein the particles have bonded therein or thereon both enzymes and the catalase activity, expressed in Baker units, amounts to at least one-sixth of the glucose oxidase activity, expressed in Sarett units.

2. Process as claimed in claim 1, characterised in that the reaction is carried out at temperatures between the freezing point of the reaction liquid and 10°C.

3. Process as claimed in one of claims 1 and 2, characterised in that 500 to 5000 Sarett units of glucose oxidase and 200 to 2000 Baker units of catalase are used, per litre of reaction liquid, in particle-bonded form.

4. Process as claimed in claim 1, characterised in that, in addition to glucose oxidase and catalase, other enzymes are also used, in soluble or immobilised form.

5. Process as claimed in one of claims 1 to 3, characterised in that it is carried out in a reactor with a stirrer, said reactor having in its lower portion, preferably at the bottom, a suction filtering device covered by a screen, membrane or filter, through which the particle-free reaction liquid is suction filtered, this liquid then being recirculated into the reactor by spraying it against the walls in the top chamber of the reactor.

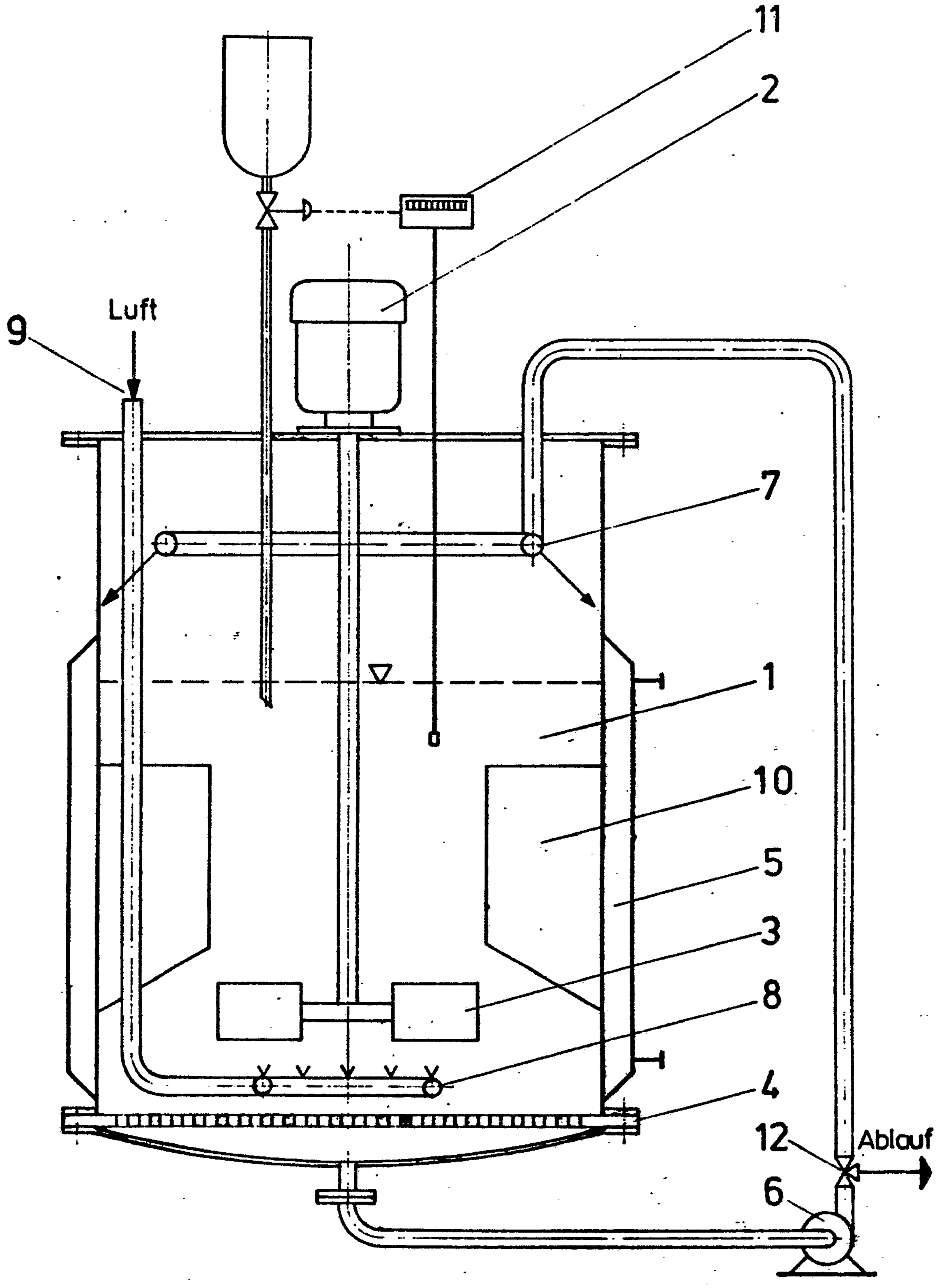


Abbildung 1